# FASCICULE DE BREVET EUROPEEN

(11) **EP 4 404 739 B1**
(45) Date de publication et mention de la délivrance du brevet: **13.05.2026**
(21) Numéro de dépôt: 22789647.9
(22) Date de dépôt: 19.09.2022
(51) Int. Cl.: A01K 43/04, A01K 45/00, G01N 22/00, G01N 33/08, A01K 43/00

(54) **DISPOSITIF POUR LE CONTROLE SANS CONTACT D'OEUFS**
VORRICHTUNG ZUR BERÜHRUNGSLOSEN ÜBERPRÜFUNG VON EIERN
DEVICE FOR CONTACTLESS INSPECTION OF EGGS

(30) Priorité: 22.09.2021 FR 2109994
(43) Date de publication de la demande: 31.07.2024
(73) Titulaire: Egg-Chick Automated Technologies, 29400 Landivisiau (FR)
(72) Inventeur: BERIER, Frédéric, 29400 LANDIVISIAU (FR); TRUBUIL, Laura, 29400 LANDIVISIAU (FR); REVOIS, Hugo, 29400 LANDIVISIAU (FR)
(74) Mandataire: Plasseraud IP
(86) Numéro de dépôt international: PCT/FR2022/051758
(87) Numéro de publication internationale: WO 2023/047047

(56) Documents cités:
- WO-A1-2010/018127
- WO-A2-2014/173831
- FR-A1- 3 089 298
- US-A1- 2020 163 314

## Description

### Domaine technique

La présente invention concerne un dispositif pour le contrôle automatique sans contact d'œufs placés dans des contenants sur une ligne de traitement.

### Technique antérieure

Il est connu dans le domaine de l'aviculture, en particulier dans la production de poussins, d'utiliser les propriétés optiques des œufs pour discriminer ces derniers et ignorer lors du traitement les œufs identifiés comme étant non susceptibles d'éclore et de donner naissance à un poussin.

Ces derniers sont essentiellement des œufs infertiles ou des œufs fécondés, mais dont l'embryon de l'œuf est mort ou encore malformé.

Cette discrimination est rendue nécessaire pour minimiser les pertes de vaccins lors de traitement in ovo, c'est-à-dire lors d'une injection par aiguille de vaccin au travers de la coquille de l'œuf en vue de favoriser son développement après éclosion et de prévenir l'apparition de maladies. Elle l'est aussi pour éviter l'explosion d'œufs pourris susceptibles de contaminer des œufs environnants qui sont viables dans le contenant, et le matériel d'injection pouvant servir à injecter ces œufs viables, ce qui risquerait également de contaminer ces derniers.

Notons que l'explosion d'œufs pourris est également susceptible de venir salir les écrans de protection des optiques utilisées dans la discrimination des œufs, le procédé associé à cette dernière étant communément appelé « mirage ».

Or, ces salissures peuvent nuire à la qualité de détection de l'état de certains œufs lorsqu'elles restent légères, voire empêcher une telle détection si les salissures sont plus importantes. La machine mise en œuvre pour réaliser le mirage, dénommée machine de mirage, doit dès lors être arrêtée pour assurer son nettoyage.

On observe en outre que ce procédé de mirage est sensible à l'environnement extérieur, des sources lumineuses telles que la lumière du soleil ou un éclairage halogène, étant susceptibles de perturber les mesures obtenues lors de l'étape de mirage des œufs contenus dans un panier.

Ce procédé est également sensible à l'état de salissure des œufs mesurés.

En outre, un tel procédé de mirage n'autorise qu'une faible cadence de traitement.

Par ailleurs, on connaît des procédés pour identifier des œufs disposés à l'envers dans un lot d'œufs.

Une telle détection des œufs en position retournée est nécessaire pour éviter lors d'une injection in ovo d'un vaccin ou de nutriments que l'aiguille de l'injecteur n'étant alors plus dirigée vers la chambre à air de l'œuf correspondant, l'embryon ne soit endommagé, voire tué.

Par exemple, un procédé de l'état de l'art pour identifier des œufs disposés à l'envers dans un lot d'œufs consiste à chauffer ce lot d'œufs avec une source de rayonnement.

On image ensuite thermiquement les œufs tandis qu'ils ne sont plus exposés à la source de rayonnement, et on analyse les images thermiques ainsi capturées pour détecter la présence d'une zone chaude et identifier les œufs disposés à l'envers, ou encore en position retournée.

En effet, la chambre à air d'un œuf agissant comme un isolant, lorsqu'un œuf placé à l'envers est soumis à un rayonnement thermique, la température de sa coquille autour de cette chambre à air augmente. Au contraire, lorsque l'œuf est à l'endroit, la chaleur générée par l'exposition de sa coquille à un rayonnement thermique se dissipe dans les liquides présents à l'intérieur de l'œuf et la coquille de ce dernier apparaît alors « froide ».

Or, un tel procédé nécessite une étape de chauffe préalable des œufs qui est consommatrice de temps et diminue les cadences de traitement susceptibles d'être atteintes sur la ligne de traitement, celles-ci n'excédant typiquement pas 70 000 œufs/h.

De plus, cette étape de chauffe préalable est typiquement réalisée avec des lampes flash de type halogène d'une puissance supérieure à 3000 W, voire 5000 W et plus, pour fournir une élévation de température détectable de la coquille de l'œuf placé à l'envers, sans significativement chauffer le reste de cet œuf. En effet, l'augmentation de température des autres parties de l'œuf, c'est-à-dire jaune, fluide amniotique et embryon, doit rester négligeable.

Or, la consommation énergétique de ces sources lumineuses est très importante et donc coûteuse.

FR 3 089 298 A1 décrit un dispositif de sexage embryonnaire aviaire par spectroscopie radiofréquence.

US 2020/163314 A1 décrit un dispositif de sexage embryonnaire aviaire et détermination de la fertilité par spectroscopie radiofréquence.

Il existe donc un besoin pressant pour un procédé de contrôle d'œufs placés dans les alvéoles de contenants, dont la conception originale permette de surmonter les inconvénients de l'art antérieur exposés ci-dessus.

### Objet de l'invention

La présente invention vise à pallier les inconvénients de l'art antérieur en proposant un dispositif de contrôle sans contact d'œufs placés dans des contenants, simples dans leur conception et dans leur mode opératoire, économiques et insensibles à l'environnement extérieur comme à l'état de propreté des œufs à mesurer.

Un autre objet de la présente invention est un tel dispositif de contrôle sans contact autorisant des cadences élevées, et à titre illustratif, supérieures à 90 000 œufs à l'heure.

Encore un objet de la présente invention est un tel dispositif de contrôle sans contact plus sûrs pour les embryons des œufs, et par conséquent, favorisant l'éclosion de ces œufs pour assurer des rendements supérieurs.

### Exposé de l'invention

L'invention est exposée dans le jeu de revendications joint.

Un procédé pour le contrôle sans contact d'un œuf comprend les étapes suivantes:
a) émettre un signal radiofréquence à ondes millimétriques à partir d'un émetteur vers un œuf,
b) détecter un signal radiofréquence à ondes millimétriques réfléchi par ledit œuf au moyen d'un capteur, ledit capteur étant placé à distance dudit œuf,
c) analyser l'intensité du signal radiofréquence à ondes millimétriques réfléchi en fonction de la distance parcourue par le signal réfléchi et comparer l'écho-radar ainsi obtenu avec un ou plusieurs échos-radars de référence représentatifs chacun d'un état de l'œuf pour en déduire son état actuel.

La conception originale de ce procédé de contrôle sans contact d'œufs d'un lot ou d'un contenant, ou encore plateau, qui met en œuvre des ondes radars dans le domaine de fréquence des ondes millimétriques autorise des cadences extrêmement rapides, typiquement supérieures à 90 000 œufs à l'heure et plus.

Ce procédé de contrôle est de plus insensible à l'environnement extérieur, par exemple à des lumières parasites, à des sources de chaleur parasites ou encore à des variations de température de l'environnement.

Ce procédé de contrôle autorise, quel que soit l'état de propreté de l'œuf à contrôler, la détermination :
- du positionnement à l'endroit ou à l'envers de l'œuf, laquelle est fiable et rapide, ou
- de l'état viable ou non de l'œuf, cette détermination nécessitant que l'œuf soit incubé puisqu'elle est basée sur la détection d'un mouvement éventuel de l'embryon. Cette mesure est basée sur la mesure de faible variation de la phase du signal réfléchi par l'embryon à l'intérieur de l'œuf. La mesure des fluctuations de phase est une fonction d'analyse des échos radars intégrée au capteur mis en œuvre.

Selon un mode de réalisation de ce procédé de contrôle sans contact d'un œuf, l'émetteur et le capteur sont positionnés à la même distance, ou sensiblement à la même distance, de l'œuf en étant disposés coaxialement. Alternativement, il est bien entendu possible que l'émetteur et le capteur ne soient pas placés à la même distance de la cible.

Selon ce procédé de contrôle sans contact d'un œuf, l'œuf étant dans une position fixe déterminant une première extrémité et une seconde extrémité dudit œuf, l'œuf ayant une chambre à air qui peut être placée soit à ladite première extrémité soit à ladite seconde extrémité, une de ces extrémités déterminant une disposition à l'envers, ou retournée, de l'œuf lorsque la chambre à air est placée à cette extrémité, on détecte la position de l'œuf pour identifier une éventuelle disposition à l'envers de cet œuf.

Alternativement, on détermine un état viable, non viable i.e. non fécondé ou mort, incertain de l'œuf ainsi analysé ou encore une absence d'œuf.
Avantageusement, on cherche à déterminer si pour chaque œuf fécondé comportant un embryon, cet embryon est vivant ou mort ou si encore, il est malformé ou trop petit au regard de son âge.
Un tel état étant alors détecté, l'œuf correspondant sera avantageusement ignoré dans la suite du traitement du contenant, notamment dans une étape d'injection sélective des œufs.
Si l'alvéole du plateau, ou contenant, est vide, on ignora également cette alvéole dans la suite du traitement du contenant.

Selon encore un autre mode de réalisation de ce procédé de contrôle sans contact d'un œuf, l'œuf étant placé dans une alvéole d'un plateau transporté par un convoyeur, ledit émetteur est agencé de sorte que ledit convoyeur déplace ledit œuf sous ledit, ou au-dessus, dudit émetteur capable d'émettre un signal radiofréquence à ondes millimétriques, ledit émetteur étant centré ou sensiblement centré sur ladite alvéole recevant l'œuf à analyser.
Bien entendu, et à titre purement illustratif, un premier émetteur peut être agencé pour venir au-dessus d'un œuf, tandis qu'un second émetteur est agencé pour venir au-dessous du même œuf afin d'acquérir deux échos radars qui seront analysés afin d'avoir une mesure plus précise.

Les ondes millimétriques correspondent à la gamme des fréquences entre 30 GHz et 300 GHz.

Selon encore un autre mode de réalisation de ce procédé de contrôle sans contact d'un œuf, à l'étape a), on émet un signal radiofréquence à ondes millimétriques dans la gamme des fréquences comprises entre 150 et 300 GHz, et de manière encore plus préférentielle entre 200 et 300 GHz.

La mise en œuvre de fréquences élevées permet d'améliorer la précision des mesures.

On pourrait alternativement utiliser une fréquence comprise entre 50 et 70 GHz, et encore mieux à une fréquence de, ou autour de, 60 GHz.

Ces fréquences subissent avantageusement un fort affaiblissement en propagation qui est proportionnel à la fréquence. De plus, et de manière avantageuse, une fréquence de 60 GHz (longueur d'onde de 5 mm) est très peu utilisée et absente du diagramme électromagnétique environnementale. Aucune pollution externe n'est susceptible de venir perturber les mesures radars réalisées.

Selon encore un autre mode de réalisation de ce procédé de contrôle sans contact d'un œuf, à l'étape a), on émet un faisceau focalisé d'ondes millimétriques sur ledit œuf.
De préférence, on utilise une lentille de focalisation et encore mieux une lentille convexe. Cette lentille convexe peut avantageusement être obtenue par impression tridimensionnelle.
De manière avantageuse, on positionne cette lentille de focalisation par rapport à l'émetteur de sorte que la divergence du faisceau d'ondes millimétriques émis par cet émetteur est inférieure strictement à 10°, et encore mieux strictement inférieure à 8° et est de préférence inférieure ou égale à 6°.

Selon encore un autre mode de réalisation de ce procédé de contrôle sans contact d'un œuf, la détection du signal radiofréquence à ondes millimétriques réfléchi par l'œuf est réalisée en l'absence d'une transmission d'un signal radiofréquence à ondes millimétriques vers ledit œuf.
On évite ainsi de mélanger le signal radiofréquence incident et le signal radiofréquence réfléchi par l'œuf.
Dans un mode de réalisation alternatif, on pourra mettre en œuvre un capteur qui fonctionne en émission continue, avec un moyen de détection interférométrique pour l'analyse du retard et du déphasage de l'écho radar.

Selon encore un autre mode de réalisation de ce procédé de contrôle sans contact d'un œuf, on réalise une étape supplémentaire de marquage des œufs non viables et disposés à l'envers et/ou une étape supplémentaire de réorientation des œufs disposés à l'envers.

Selon encore un autre mode de réalisation de ce procédé de contrôle sans contact d'un œuf, on traite les données à l'étape c) liées à un contenant donné, les informations obtenues par ce traitement étant stockées et/ou adressées à un poste de traitement des œufs distant tel qu'un dispositif d'administration par injection in ovo d'œufs, de sorte que ce poste de traitement distant recevant ledit contenant d'œufs à traiter dispose des informations nécessaires à son traitement. On assure ainsi une continuité dans le traitement de chaque contenant, le poste de traitement suivant sur la ligne de traitement à hautes cadences ayant déjà reçu du dispositif de contrôle sans contact les informations relatives au contenant à traiter avant sa prise en charge.

Selon encore un autre mode de réalisation de ce procédé de contrôle, l'acquisition des échos-radars pour un contenant et le traitement des données liées à des échos-radars sont réalisés en parallèle de sorte que le traitement des données liées à des échos-radars obtenus pour un premier contenant est réalisé tandis qu'on acquiert des échos-radars pour un contenant suivant immédiatement adjacent sur ledit convoyeur

Selon encore un autre mode de réalisation de ce procédé de contrôle sans contact d'un œuf, lesdits plateaux sont transportés à une vitesse constante V par un convoyeur rectiligne.
Dans la mesure où chaque contenant se déplace en translation, en particulier à vitesse constante sur un convoyeur rectiligne ce qui, à grande cadence, a l'avantage d'éviter les à-coups, les œufs restent stables dans leur alvéole respective du contenant et par conséquent, présentent un positionnement optimal pour leur injection subséquente.
A titre d'exemple, il s'agit d'un convoyeur à bande sans fin.

Selon encore un autre mode de réalisation de ce procédé de contrôle sans contact d'un œuf, lesdits plateaux sont transportés à une vitesse constante strictement supérieure ou égale à 1 m/min, et encore mieux à 10 m/min, et de manière encore plus préférentielle de l'ordre de 15 m/min, en étant espacés d'une distance de sécurité au moins égale à d = 100 mm pour assurer une cadence de traitement élevée.

Selon encore un autre mode de réalisation de ce procédé de contrôle sans contact d'un œuf, pendant la mesure des œufs contenus dans ledit contenant, on détermine au moyen d'un capteur de position, la longueur dudit contenant en cours de mesure, on compare la longueur ainsi mesurée dudit contenant avec sa longueur réelle et on en déduit l'absence ou l'existence d'un déplacement intempestif dudit contenant pendant l'étape a) et/ou l'étape b) du procédé de contrôle sans contact.
De manière avantageuse, la conception originale de cette étape autorise une détection simple et peu onéreuse d'un mouvement intempestif d'un contenant transporté par un convoyeur, ce mouvement résultant en une perte de sa position exacte sur le convoyeur pendant une mesure des œufs transportés par ce dernier.

De manière préférentielle, ce capteur de position étant agencé pour détecter les extrémités avant et arrière d'un contenant en déplacement sur ledit convoyeur, on mesure l'intervalle de temps séparant la détection par ce capteur desdites extrémités et on calcule une longueur mesurée du contenant par le produit de cet intervalle de temps par la vitesse d'entrainement de ce contenant le long de ladite ligne de traitement.
De manière avantageuse, ce capteur de position est agencé pour détecter ces extrémités d'un contenant lorsqu'elles passent au droit de ce capteur lors du transport du contenant le long de la ligne de traitement.

Alternativement, ce capteur de position étant agencé pour détecter les extrémités avant et arrière d'un contenant en déplacement sur ledit convoyeur, on détermine le nombre de points codeur écoulés entre la détection de ces extrémités avant et arrière par ledit capteur et on convertit ce nombre de points codeur en longueur mesurée dudit contenant.
La distance parcourue par la bande du convoyeur pendant un tour codeur étant connue, le nombre de points codeur ainsi déterminé peut aisément être traduit ou converti en distance.
Rappelons que le nombre de points codeur par tour codeur est lié à la résolution de ce codeur.
Le codeur émet avantageusement un signal électrique donnant le nombre de points codeur réalisés entre la détection des deux extrémités avant et arrière.
De manière avantageuse, cette mesure de la longueur du contenant est ainsi indépendante de la vitesse d'entraînement du convoyeur

Lors de l'étape de comparaison, on peut également tenir compte d'une plage de tolérance préalablement déterminée sur la longueur mesurée du contenant.

Selon encore un autre mode de réalisation de ce procédé de contrôle sans contact d'un œuf, à l'étape c) et avant comparaison, on réalise une étape de traitement pour supprimer d'éventuels signaux parasites dans l'écho-radar obtenu.
Avantageusement, on ne conserve ainsi que le signal utile provenant de l'œuf ainsi contrôlé.
Ces signaux parasites peuvent provenir du contenant pour lequel les picots hauts/ les bords épais peuvent ressortir à l'écho radar.
Différentes méthodes de traitement peuvent être envisagées pour supprimer ces signaux parasites, telles que
- réduction de la zone de données de l'écho-radar utile pour la classification : par exemple, en ne gardant que les échos de profondeurs utiles (moitié haute de l'œuf), ou en supprimant les échantillons des bords de l'œuf.
- réaliser une mesure d'un "contenant, ou plateau, vide" et prendre le signal ainsi obtenu comme référence à "soustraire" aux signaux acquis lorsque l'on scanne des œufs.

La présente invention concerne un dispositif pour le contrôle automatique sans contact d'œufs, tels que des œufs de volatiles, comprenant pour chaque œuf, un module radar configuré pour émettre des ondes millimétriques vers ledit œuf et détecter des ondes millimétriques qui sont réfléchies par ledit œuf, ledit module radar émettant des signaux de sortie à partir desdites ondes millimétriques réfléchies ainsi détectées, ledit dispositif de mesure comprenant une unité de traitement pour analyser lesdits signaux de sortie et en déduire un état le positionnement à l'endroit ou à l'envers de l'œuf correspondant, ou encore un état viable, non viable ou incertain de l'œuf correspondant.

Un tel appareil permet avantageusement le contrôle sans contact, d'œufs disposés dans un contenant, ou panier, tout en étant plus sûr pour les embryons des œufs.

Cet appareil est particulièrement adapté pour le traitement à haute cadence d'objets, sur une ligne automatique industrielle de traitement d'objets ayant un contenu fragile.

Selon un mode de réalisation de ce dispositif de contrôle sans contact d'œufs, chaque module radar comprend une lentille pour focaliser le faisceau d'ondes millimétriques sur l'œuf correspondant, ladite lentille de focalisation étant de préférence une lentille convexe.

Selon un autre mode de réalisation de ce dispositif de contrôle sans contact d'œufs, ledit module radar est configuré pour envoyer vers l'œuf un signal radiofréquence à ondes millimétriques dans la gamme des fréquences comprises entre 150 et 300 GHz, et de manière encore plus préférentielle entre 200 et 300 GHz.

Selon l'invention, chaque module radar est configuré pour émettre un faisceau d'ondes millimétriques ayant une puissance inférieure à 0,15 mW/cm², et encore mieux inférieure ou égale à 0,1 mW/cm² pour éviter tout risque pour le développement de l'embryon.

Selon encore un autre mode de réalisation de ce dispositif de contrôle sans contact d'œufs, ledit module radar comprend une première antenne pour émettre un faisceau d'ondes millimétriques vers ledit œuf et une seconde antenne pour recevoir les ondes millimétriques réfléchies par ledit œuf, lesdites première et seconde antennes étant portées par un même support en étant coaxiales.

Selon encore un autre mode de réalisation de ce dispositif de contrôle sans contact d'œufs, il comprend un convoyeur rectiligne pour déplacer des plateaux comportant des alvéoles disposées en rangées et colonnes, chaque rangée comprenant n alvéoles, ledit convoyeur définissant un axe de convoyage, ledit dispositif comportant n modules radars alignés le long d'un même axe de mesure qui est perpendiculaire, ou sensiblement perpendiculaire, audit axe de convoyage, lesdits modules radars étant espacés les uns des autres d'une distance égale ou sensiblement égale pour venir chacun au-dessus, et/ou en dessous, d'une seule des alvéoles de ladite rangée lorsque cette dernière est placée sous lesdits, respectivement et/ou au-dessus desdits, modules radars.

Avantageusement, ce dispositif de contrôle comporte une unité de commande contrôlant la vitesse de transport des plateaux par le convoyeur, laquelle est configurée pour définir une vitesse de transport constante de ces plateaux le long d'un chemin de convoyage.
De manière plus générale, le déplacement des plateaux sur le convoyeur rectiligne est réalisé sans à-coups.
Un déplacement à vitesse constante garantit notamment la stabilité des œufs dans leur alvéole et par conséquent, une orientation optimale de ces œufs pour leur traitement ultérieur sur d'autres postes d'une ligne de traitement à haute cadence.

De préférence, le dispositif comprend également au moins un capteur de position placé en amont desdits modules radars sur ledit convoyeur et relié à une unité centrale de manière à lancer un cycle d'acquisition de données pour un plateau d'œufs dont l'extrémité avale est détectée en une première position définie par ledit capteur de position, ladite unité centrale étant configurée pour déclencher lesdites émissions d'ondes millimétriques à chaque passage d'une rangée du plateau d'œufs en cours d'acquisition.

De manière avantageuse, chaque capteur de position est une cellule photoélectrique qui est, par exemple, placée sur le côté de la bande transporteuse du convoyeur.

Selon encore un autre mode de réalisation de ce dispositif de contrôle sans contact d'œufs, chaque module radar est agencé pour être centré, ou sensiblement centré, sur l'axe de symétrie de l'alvéole correspondante lorsque cette alvéole du plateau en cours d'acquisition passe sous ledit, respectivement et/ou au-dessus d'un, module radar.

Le dispositif de contrôle sans contact d'œufs peut ainsi comporter pour chaque alvéole correspondante, un premier module radar destiné à être placé au-dessus de l'alvéole et un second module radar destiné à être positionné simultanément en-dessous de cette alvéole lorsque le plateau se déplace entre ces modules radars.

Selon encore un autre mode de réalisation de ce dispositif de contrôle sans contact d'œufs, il comprend un module de communication pour adresser des données ou informations obtenues par traitement des échos-radars des œufs d'un plateau donné à un poste distant tel qu'un dispositif d'injection in ovo des œufs de ce plateau.

Selon encore un autre mode de réalisation de ce dispositif de contrôle sans contact d'œufs, il comprend des moyens de marquage des œufs non viables et de ceux disposés à l'envers. Il peut comprendre également un dispositif assurant la réorientation des œufs disposés à l'envers afin de les remettre à l'endroit dans leur alvéole.

### Brève description des dessins

D'autres avantages, buts et caractéristiques particulières de la présente invention ressortiront de la description qui va suivre, faite, dans un but explicatif et nullement limitatif, en regard des dessins annexés, dans lesquels :
**Fig. 1**

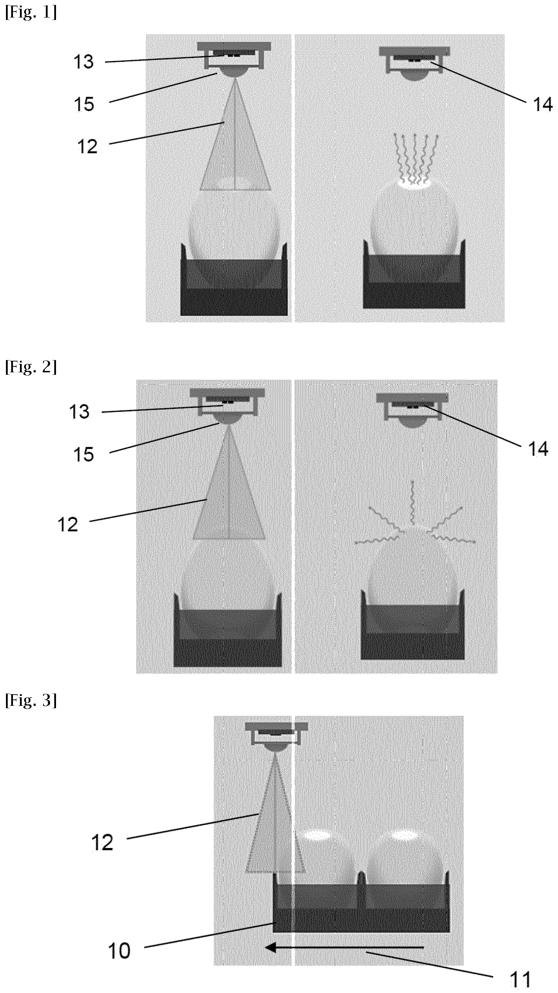

   [Fig. 1] est une représentation schématique partielle d'un dispositif pour le contrôle sans contact d'œufs disposés dans des paniers transportés par un convoyeur selon un mode de réalisation particulier de la présente invention, l'œuf étant ici positionné à l'endroit dans son alvéole ;
**Fig. 2**
   [Fig. 2] montre la détection d'un œuf positionné à l'envers, ou étant retournée, dans son alvéole avec le dispositif pour le contrôle sans contact d'œufs illustré à la Fig. 1 ;
**Fig. 3**
   [Fig. 3] est une vue schématique et partielle d'un plateau défilant sous un module radar du dispositif pour le contrôle sans contact d'œufs de la Fig. 1, le déclenchement de la mesure étant réalisé lorsque le bord avant de l'alvéole recevant l'œuf à mesurer passe au droit du module radar ;
**Fig. 4**

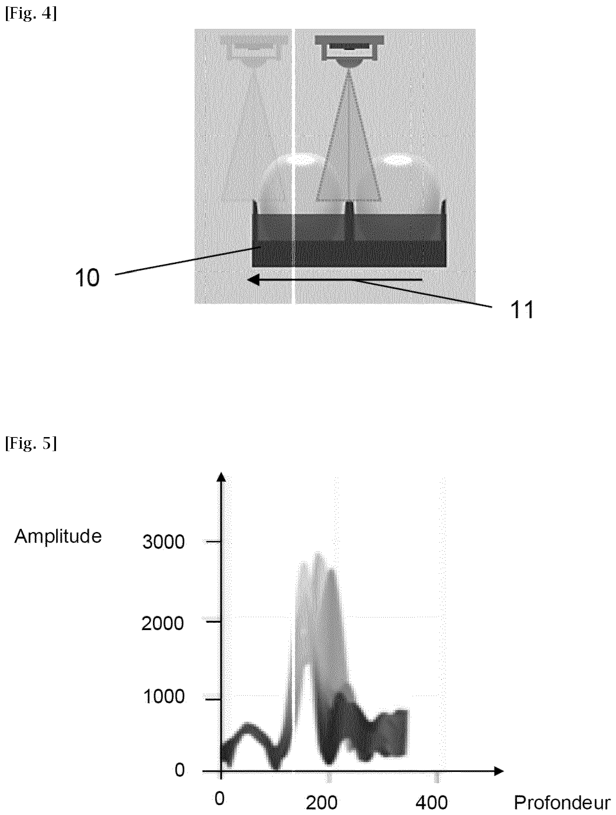

   [Fig. 4] est une vue schématique et partielle du plateau de la Fig.3 à un instant ultérieur dans son transport sur le convoyeur, l'arrêt de la mesure étant réalisé lorsque le bord arrière de l'alvéole recevant l'œuf à mesurer passe au droit du module radar ;
**Fig. 5**
   [Fig. 5] est un diagramme temporel obtenu avec le dispositif pour le contrôle sans contact d'œufs illustré à la Fig. 1 pour un œuf positionné à l'endroit dans son alvéole ;
**Fig. 6**

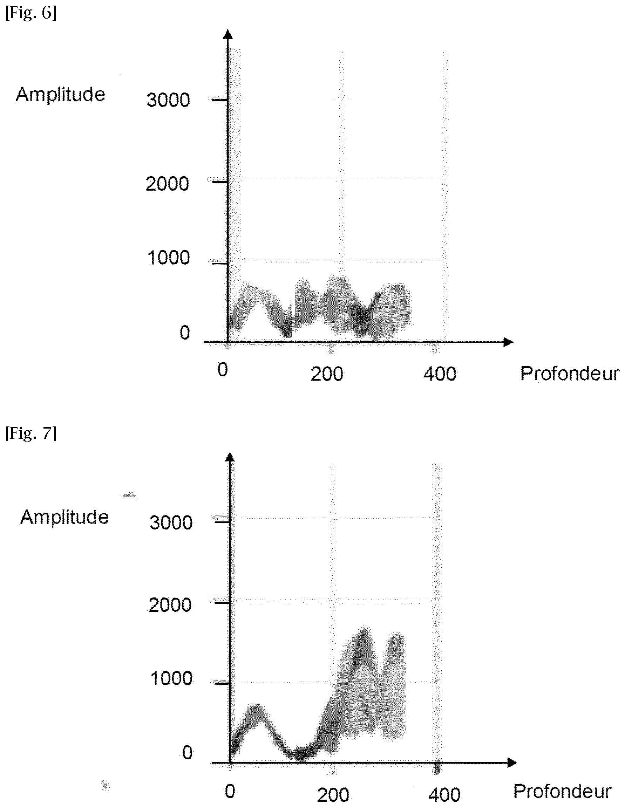

   [Fig. 6] est un diagramme temporel obtenu avec le dispositif pour le contrôle sans contact d'œufs illustré à la Fig. 1 pour un œuf positionné à l'envers dans son alvéole ;
**Fig. 7**
   [Fig. 7] est un diagramme temporel obtenu avec le dispositif pour le contrôle sans contact d'œufs illustré à la Fig. 1 en l'absence d'œuf dans l'alvéole ;
**Fig. 8**

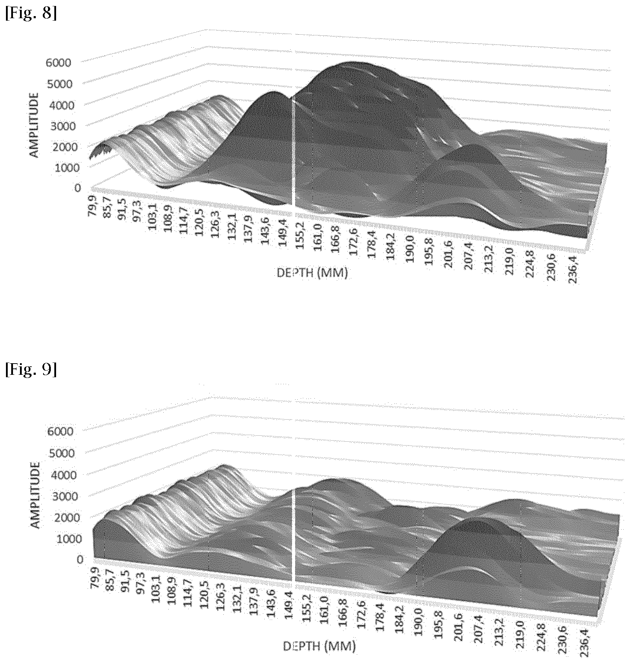

   [Fig. 8] montre un ensemble de mesures réalisées avec le dispositif pour le contrôle sans contact d'œufs illustré à la Fig. 1, pour quinze œufs au quatorzième jour, les œufs étant positionnés à l'endroit dans leur alvéole respective ;
**Fig. 9**
   [Fig. 9] montre un ensemble de mesures réalisées avec le dispositif pour le contrôle sans contact d'œufs illustré à la Fig. 1, pour quinze œufs au quatorzième jour, les œufs étant positionnés à l'envers, ou encore étant retournée, dans leur alvéole respective ;

### Description des modes de réalisation

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

Tout d'abord, on note que les figures ne sont pas à l'échelle.

Les Figures 1 à 4 illustrent de manière schématique un dispositif pour le contrôle sans contact d'œufs disposés dans des paniers 10 en défilement sur un convoyeur rectiligne 11 selon un mode de réalisation particulier de la présente invention.

Ce convoyeur rectiligne 11 qui est ici de type à bande sans fin, comporte une unité de commande (non représentée) contrôlant la vitesse de transport des paniers 10.

De manière avantageuse, ces paniers 10 sont déplacés à vitesse constante pour éviter la génération d'à-coups susceptibles d'induire des mouvements des œufs et/ou des chocs aux embryons de ces œufs.

Ces paniers 10 en défilement qui présentent une forme générale "rectangulaire", comprennent une pluralité d'alvéoles, ou cellules, dans chacune desquelles est normalement reçu un œuf. Ces alvéoles sont réparties en rangées et colonnes, chaque rangée comprenant ici dix (10) alvéoles.

Ces paniers 10 sont avantageusement réalisées dans une matière transparente aux ondes millimétriques, telle qu'une matière plastique.

De préférence, les alvéoles de ces paniers 10 présentent une forme évasée en ayant leur extrémité supérieure ouverte le plus large possible de sorte que les bords de cette ouverture ne rencontrent pas le faisceau d'ondes millimétriques 12 envoyé vers l'œuf correspondant.

Ces œufs sont préférablement orientés dans leur alvéole en vue de leur injection in ovo, c'est-à-dire que leur extrémité la plus étroite est disposée vers le bas pour que la chambre à air soit disposée vers le haut. Cette position de l'œuf est considérée comme étant « à l'endroit ». Les risques sont ainsi moindres d'endommager l'embryon de l'œuf par l'aiguille d'injection. L'œuf est, de préférence, orienté verticalement dans son alvéole. Il arrive cependant que des œufs soient mal positionnés, ou soient retournées, dans leur alvéole respective. Si l'œuf est retourné (i.e. avec sa chambre à air vers le bas), on parle d'œuf disposé « à l'envers ».

Le dispositif décrit dans ce document permet de contrôler l'orientation des œufs de manière très simple et rapide.

Ce dispositif de contrôle sans contact comporte dix modules radars, un par alvéole d'une rangée du panier 10, ces modules radars étant alignés en étant espacés les uns des autres de sorte qu'un seul module radar se trouve au-dessus d'une alvéole à la fois. De préférence, on s'assurera que chaque module radar de la rangée correspondante est centré ou sensiblement centré sur son alvéole en vue de réaliser une mesure. Notons qu'il n'est pas nécessaire que la chambre à air de l'œuf soit centrée par rapport au module radar. Toutefois, le signal des ondes millimétriques réfléchies par l'œuf est maximal dans la configuration centrée de la chambre à air.

Ce module radar comprend une première antenne 13 pour émettre un faisceau d'ondes millimétriques 12, à une fréquence de 60 GHz, vers l'œuf correspondant. Il comporte également une seconde antenne 14 pour recevoir les ondes millimétriques réfléchies par cet œuf.

Ces première et seconde antennes 13, 14 sont portées par un même support en étant disposées coaxialement. Ce module radar comprend également une lentille convexe 15 pour focaliser le faisceau d'ondes millimétriques 12 sur l'œuf correspondant.

De manière avantageuse, la divergence du faisceau d'ondes millimétriques 12 envoyé vers ledit œuf est de l'ordre de 6° pour rencontrer seulement l'œuf.

C'est la forme de l'interface liquide/air qui en réfléchissant les ondes millimétriques va permettre d'identifier le positionnement à l'endroit ou à l'envers de l'œuf correspondant dans son alvéole. La coquille est considérée comme transparente à ces fréquences entre 30 GHz et 300 GHz.

L'onde est réfléchie dans tous les cas par la surface du liquide amniotique, ou du liquide allantoïque en fonction du stade de développement. Le faisceau d'ondes millimétriques 12 incident serait réfléchi sans déformation si la surface liquide était plane. Elle serait alors non dispersée. L'intensité mesurée en retour serait uniquement dépendante de la distance entre l'œuf et la seconde antenne 14 de détection et de la section de l'œuf.

Ainsi, et comme illustré sur la Fig.1, lorsque l'œuf est en position à l'endroit dans son alvéole, il montre sa partie arrondie et la surface du liquide est concave. L'intensité du signal lié à la détection du faisceau d'ondes millimétriques réfléchi par l'œuf est grande.

Lorsque la surface du liquide amniotique, ou du liquide allantoïque, est convexe (Fig. 2), le faisceau d'ondes millimétriques 12 incident est dispersé et par conséquent l'intensité du signal lié à la détection du faisceau d'ondes millimétriques réfléchi, qui revient sur la seconde antenne 14, est amoindrie. L'œuf est disposé à l'envers, ou est encore retourné.

Les paniers 10 sont alimentés sur le convoyeur rectiligne 11 à intervalle minimal régulier en étant alignés sur une file. Ils présentent ainsi un espacement minimal entre eux.

Les Figures 5 à 7 illustrent des exemples de diagrammes temporels réalisés par le dispositif de contrôle sans contact d'œufs illustré aux Figures 1 à 4.

Grâce à la signature radar de chaque œuf, il est possible de déterminer de manière très fiable l'orientation de cet œuf dans son alvéole (Figs. 5 et 6), voire l'absence d'œuf dans l'alvéole correspondante (Fig. 7).

Cette détermination est réalisée par comparaison de l'écho radar mesuré pour l'œuf à contrôler avec des échos radars de référence qui ont été préalablement enregistrés dans une bibliothèque de données stockée sur une unité de stockage.

Par exemple, le diagramme temporel illustré à la Fig. 5 correspond à un œuf bien positionné dans son alvéole tandis que le diagramme temporel représenté à la Fig. 6 correspond à un œuf positionné à l'envers, ou encore retourné, dans son alvéole.

Il a été constaté que l'identification de la position à l'envers ou à l'endroit de l'œuf dans son alvéole au moyen du dispositif de contrôle sans contact décrit précédemment est très aisée.

Des taux d'identification de l'ordre de 100% sont atteints montrant l'intérêt de la présente invention.

## Revendications

1. Dispositif pour le contrôle automatique sans contact d'une pluralité d'œufs, tels que des œufs de volatiles, comprenant pour chaque œuf, un module radar (13, 14) configuré pour émettre des ondes millimétriques vers ledit œuf et détecter des ondes millimétriques qui sont réfléchies par ledit œuf, ledit module radar émettant des signaux de sortie à partir desdites ondes millimétriques réfléchies ainsi détectées, ledit dispositif de mesure comprenant une unité de traitement pour analyser lesdits signaux de sortie et en déduire le positionnement à l'endroit ou à l'envers de l'œuf correspondant, ou encore un état viable, non viable ou incertain de l'œuf correspondant, **caractérisé en ce que** chaque module radar est configuré pour émettre un faisceau d'ondes millimétriques ayant une puissance inférieure à 0,15 mW/cm², et encore mieux inférieure ou égale à 0,1 mW/cm² pour éviter tout risque pour le développement de l'embryon.

2. Dispositif selon la revendication 1, **caractérisé en ce que** chaque module radar (13, 14) comprend une lentille (15) pour focaliser le faisceau d'ondes millimétriques sur l'œuf correspondant, ladite lentille (15) de focalisation étant de préférence une lentille convexe.

3. Dispositif selon la revendication 1 ou 2, **caractérisé en ce que** ledit module radar (13, 14) est configuré pour envoyer vers l'œuf un signal radiofréquence à ondes millimétriques dans la gamme de fréquences comprises entre 150 et 300 GHz, et de manière encore plus préférentielle entre 200 et 300 GHz.

4. Dispositif selon l'une quelconque des revendications 1 à 3, **caractérisé en ce que** ledit module radar comprend une première antenne (12) pour émettre un faisceau d'ondes millimétriques vers ledit œuf et une seconde antenne (14) pour recevoir les ondes millimétriques réfléchies par ledit œuf, lesdites première et seconde antennes (14) étant portées par un même support en étant coaxiales.

5. Dispositif selon l'une quelconque des revendications 1 à 4, **caractérisé en ce qu'**il comprend un convoyeur (11) rectiligne pour déplacer des plateaux (10) comportant des alvéoles disposées en rangées et colonnes, chaque rangée comprenant n alvéoles, ledit convoyeur (11) définissant un axe de convoyage, ledit dispositif comportant n modules radars alignés le long d'un même axe de mesure qui est perpendiculaire, ou sensiblement perpendiculaire, audit axe de convoyage, lesdits modules radars étant espacés les uns des autres d'une distance égale ou sensiblement égale pour venir chacun au-dessus, et/ou en dessous, d'une seule des alvéoles de ladite rangée lorsque cette dernière est placée sous lesdits, respectivement et/ou au-dessus desdits, modules radars.

6. Dispositif selon la revendication 5, **caractérisé en ce qu'**il comprend un capteur de position placé en amont desdits modules radars sur ledit convoyeur (11) et relié à une unité centrale de manière à lancer un cycle d'acquisition de données pour un plateau (10) d'œufs dont l'extrémité avale est détectée en une première position définie par ledit capteur de position, ladite unité centrale étant configurée pour déclencher lesdites émissions d'ondes millimétriques à chaque passage d'une rangée du plateau (10) d'œufs en cours d'acquisition.

7. Dispositif selon la revendication 5 ou 6, **caractérisé en ce que** chaque module radar (13, 14) est agencé pour être centré, ou sensiblement centré, sur l'axe de symétrie de l'alvéole correspondante lorsque cette alvéole du plateau (10) en cours d'acquisition passe sous ledit, respectivement et/ou au-dessus d'un, module radar.

## Patentansprüche

1. Vorrichtung für die automatische berührungslose Kontrolle einer Vielzahl von Eiern, wie Geflügeleiern, umfassend für jedes Ei ein Radarmodul (13, 14), das zum Aussenden von Millimeterwellen zu dem Ei und Erfassen von Millimeterwellen konfiguriert ist, die durch das Ei reflektiert werden, wobei das Radarmodul Ausgangssignale aus den so erfassten reflektierten Millimeterwellen aussendet, die Messvorrichtung umfassend eine Verarbeitungseinheit zum Analysieren der Ausgangssignale und Ableiten der Positionierung des entsprechenden Eis richtig oder verkehrt herum oder auch eines lebensfähigen, nicht lebensfähigen oder ungewissen Zustandes des entsprechenden Eis, **dadurch gekennzeichnet, dass** jedes Radarmodul zum Aussenden eines Millimeterwellenstrahls konfiguriert ist, der eine Leistung von weniger als 0,15 mW/cm² und noch besser von weniger als oder gleich 0,1 mW/cm² zum Vermeiden jedes Risikos für die Entwicklung des Embryos aufweist.

2. Vorrichtung nach Anspruch 1, **dadurch gekennzeichnet, dass** jedes Radarmodul (13, 14) eine Linse (15) zum Fokussieren des Millimeterwellenstrahls auf das entsprechende Ei umfasst, wobei die Fokussierlinse (15) vorzugsweise eine konvexe Linse ist.

3. Vorrichtung nach Anspruch 1 oder 2, **dadurch gekennzeichnet, dass** das Radarmodul (13, 14) zum Senden eines Millimeterwellenhochfrequenzsignals in dem Frequenzbereich zwischen 150 und 300 GHz, und noch mehr bevorzugt zwischen 200 und 300 GHz an das Ei konfiguriert ist.

4. Vorrichtung nach einem der Ansprüche 1 bis 3, **dadurch gekennzeichnet, dass** das Radarmodul eine erste Antenne (12) zum Aussenden eines Millimeterwellenstrahls zu dem Ei und eine zweite Antenne (14) zum Empfangen der Millimeterwellen umfasst, die durch das Ei reflektiert werden, wobei die erste und die zweite Antenne (14) durch einen gleichen Träger getragen werden und koaxial sind.

5. Vorrichtung nach einem der Ansprüche 1 bis 4, **dadurch gekennzeichnet, dass** sie einen geradlinigen Förderer (11) zum Bewegen von Ablagen (10) umfasst, die Vertiefungen vorweisen, die in Reihen und Spalten angeordnet sind, jede Reihe umfassend n Vertiefungen, wobei der Förderer (11) eine Förderachse definiert, wobei die Vorrichtung n Radarmodule vorweist, die entlang einer gleichen Messachse ausgerichtet sind, die senkrecht oder im Wesentlichen senkrecht zu der Förderachse ist, wobei die Radarmodule zum Gelangen jeweils über und/oder unter eine einzige der Vertiefungen der Reihe, wenn diese letztere jeweils unterhalb und/oder über den Radarmodulen platziert ist, um einen gleichen oder im Wesentlichen gleichen Abstand voneinander beabstandet sind.

6. Vorrichtung nach Anspruch 5, **dadurch gekennzeichnet, dass** sie einen Positionssensor umfasst, der stromaufwärts von den Radarmodulen auf dem Förderer (11) platziert und mit einer Zentraleinheit verbunden ist, um einen Datensammelzyklus für eine Eierablage (10) zu starten, deren stromabwärtiges Ende in einer ersten Position erfasst wird, die durch den Positionssensor definiert ist, wobei die Zentraleinheit zum Auslösen der Millimeterwellenaussendungen bei jedem Durchgang einer Reihe der Eierablage (10) während des Sammelns konfiguriert ist.

7. Vorrichtung nach Anspruch 5 oder 6, **dadurch gekennzeichnet, dass** jedes Radarmodul (13, 14) angeordnet ist, um auf der Symmetrieachse der entsprechenden Vertiefung zentriert oder im Wesentlichen zentriert zu sein, wenn diese Vertiefung der Ablage (10) während des Sammelns unterhalb und/oder über einem Radarmodul hindurchgeht.

## Claims

1. Device for automatically contactlessly inspecting a plurality of eggs, such as poultry eggs, comprising for each egg a radar module (13, 14) configured to emit millimeter waves towards said egg and detect millimeter waves which are reflected by said egg, said radar module emitting output signals from said detected reflected millimeter waves, said measuring device comprising a processing unit for analyzing said output signals and deducing therefrom the right-side-up or upside-down positioning of the corresponding egg, or a viable, non-viable or uncertain state of the corresponding egg, **characterized in that** each radar module is configured to emit a beam of millimeter waves having a power of less than 0.15 mW/cm², and even more preferably less than or equal to 0.1 mW/cm² in order to avoid any risk to the development of the embryo.

2. Device according to claim 1, **characterized in that** each radar module (13, 14) comprises a lens (15) for focusing the millimeter-wave beam onto the corresponding egg, said focusing lens (15) preferably being a convex lens.

3. Device according to either claim 1 or claim 2, **characterized in that** said radar module (13, 14) is configured to send a millimeter-wave radio-frequency signal toward the egg in the frequency range of between 150 and 300 GHz, and even more preferentially between 200 and 300 GHz.

4. Device according to any of claims 1 to 3, **characterized in that** said radar module comprises a first antenna (12) for emitting a millimeter-wave beam toward said egg and a second antenna (14) for receiving the millimeter waves reflected by said egg, said first and second antennas (14) being carried by the same support while being coaxial.

5. Device according to any of claims 1 to 4, **characterized in that** it comprises a straight conveyor (11) for moving trays (10) comprising divots arranged in rows and columns, each row comprising n divots, said conveyor (11) defining a conveying axis, said device comprising n radar modules aligned along the same measurement axis that is perpendicular, or substantially perpendicular, to said conveying axis, said radar modules being spaced apart from one another by an equal or substantially equal distance to come above and/or below a single one of the divots of said row when the latter is placed below and or above, respectively, said radar modules.

6. Device according to claim 5, **characterized in that** it comprises a position sensor placed upstream of said radar modules on said conveyor (11) and connected to a central unit so as to launch a data acquisition cycle for an egg tray (10), the downstream end of which is detected in a first position defined by said position sensor, said central unit being configured to trigger said millimeter-wave emissions on each passage of a row of the egg tray (10) during acquisition.

7. Device according to either claim 5 or claim 6, **characterized in that** each radar module (13, 14) is arranged to be centered, or substantially centered, on the axis of symmetry of the corresponding divot when this divot of the tray (10) being acquired passes under, and/or above, respectively, a radar module.
